# EUROPÄISCHE PATENTSCHRIFT

(11) **EP 1 917 061 B1**
(45) Veröffentlichungstag und Bekanntmachung des Hinweises auf die Patenterteilung: **11.03.2009**
(21) Anmeldenummer: 06761265.5
(22) Anmeldetag: 08.08.2006
(51) Int. Cl.: A61M 25/00

(54) **MEDIZINTECHNISCHE VORRICHTUNG ZUM ZUMINDEST TEILWEISEN EINFÜHREN IN EINEN KÖRPERGANG**
MEDICAL DEVICE FOR AT LEAST PARTIALLY INTRODUCING INTO A BODY PASSAGE
APPAREIL MEDICAL DESTINE A ETRE INTRODUIT AU MOINS PARTIELLEMENT DANS UN PASSAGE CORPOREL

(30) Priorität: 26.08.2005 CH 14052005
(43) Veröffentlichungstag der Anmeldung: 07.05.2008
(73) Patentinhaber: Von Weymarn-Schärli, Alexander, 4059 Basel (CH)
(72) Erfinder: Von Weymarn-Schärli, Alexander, 4059 Basel (CH)
(74) Vertreter: Volpert, Marcus
(86) Internationale Anmeldenummer: PCT/CH2006/000414
(87) Internationale Veröffentlichungsnummer: WO 2007/022650

(56) Entgegenhaltungen:
- EP-A- 0 371 486
- WO-A-20/04035124
- WO-A-20/05042078
- US-A- 5 250 069
- US-A- 6 159 195
- US-A1- 2004 143 239

## Beschreibung

Die Erfindung bezieht sich auf eine medizintechnische Vorrichtung zum zumindest teilweisen Einführen in einen Körpergang nach dem Oberbegriff des Patentanspruchs 1.

Eine medizintechnische Vorrichtung zum zumindest teilweisen Einführen in einen Körpergang gemäss dem Oberbegriff des Patentanspruchs 1 ist aus der WO 2005/042078 A1 bekannt. Diese Vorrichtung hat eine Steuereinrichtung, mittels der ein Druckmedium in die Vorrichtung bzw. ein Vakuum an die Vorrichtung einleitbar bzw. anlegbar ist, wobei das Druckmedium beispielsweise in den Innenraum des Innenkörpers anlegbar sein kann. Gemäss dieser Schrift ist es auch möglich, den Innenkörper doppelwandig auszubilden und dadurch beispielsweise durch Aufblasen eine radiale Dehnung des Innenkörpers herbeizuführen.

Eine andere medizintechnische Vorrichtung ist beispielsweise aus der WO 2004/035124 A1 bekannt. Die bekannte Vorrichtung ist eine Führungseinrichtung insbesondere zum Positionieren von Kathetern in einem Körpergang. Sie hat einen schlauchartigen Innenkörper, welcher als erster Strang ausgebildet ist. Dieser ist aus einem dehnbaren, elastischen Material gefertigt und auf seiner Umfangsfläche von mehreren zweiten Strängen aus Draht unter Belassung zwickelartiger Zwischenräume dicht umgeben. Der erster Strang genannte Innenkörper kann in seinem gedehnten Zustand auf die mehreren zweiten Stränge einen radial wirkenden Druck ausüben. Dazu ist der Innenraum des Innenkörpers mit einem unter Druck stehenden Fluid, vorzugsweise einer Flüssigkeit, beaufschlagbar. Nachteilig ist dabei, dass die bekannte Vorrichtung ausschliesslich als so genannte Führungseinrichtung zum Positionieren von Kathetern einsetzbar ist.

Der Erfindung liegt die Aufgabe zugrunde, eine medizintechnische Vorrichtung der eingangs erwähnten Art zu schaffen, welche vielseitiger einsetzbar und vorteilhafter handhabbar ist.

Diese Aufgabe wird erfindungsgemäss durch eine medizintechnische Vorrichtung mit den Merkmalen des Patentanspruchs gelöst.

Erfindungsgemäss ist der Innenkörper in Form eines Doppelschlauches mit einem eine Innenwand des Innenkörpers bildenden Innenschlauch und einem eine Aussenwand des Innenkörpers bildenden, den Innenschlauch umfangsseitig konzentrisch umgebenden Aussenschlauch ausgebildet und ist die Einrichtung zum Überführen der Vorrichtung von einem flexiblen in einen versteiften Zustand und umgekehrt dadurch gebildet, dass sich der Aussenschlauch durch Erhöhung des Druckes in einem ringförmigen Zwischenraum des Innenkörpers radial dehnt und dadurch direkt oder indirekt durch weitere Mittel einen Druck auf auf den Hüllenkörper ausübt. Dadurch ist sichergestellt, dass der Innenkörper einen Innenraum hat, welcher grössenmässig festlegbar ist. So kann beispielsweise der Innendurchmesser des Innenkörpers mindestens 1 bis 5 und mehr mm betragen und daher zum Aufnehmen beispielsweise eines Katheters dienen. Die erfindungsgemässe medizintechnische Vorrichtung kann damit sowohl eine so genannte Führungseinrichtung insbesondere zum Positionieren von Kathetern in einem Körpergang oder selbst eine Vorrichtung zum zumindest teilweisen Einführen in einen Körpergang, insbesondere eine Schleuse oder ein Katheter, sein. Damit steht im Innenkörper der Vorrichtung ein definierter Raum zur Verfügung, wobei durch die Dehnfähigkeit des Aussenschlauches des Innenkörpers trotzdem eine Möglichkeit geschaffen ist, die gesamte Vorrichtung von einem weitgehend flexiblen in einen weitgehend versteiften, aber dem Bedarf angepassten gebogenen, Zustand und umgekehrt zu überführen.

Vorteilhafterweise ist der Aussenschlauch aus einem Material gefertigt, das dehnfähiger als dasjenige des Innenschlauches vorzugsweise dadurch ist, dass die Dicke des Aussenschlauches geringer als diejenige des Innenschlauches ist. Damit ist gewährleistet, dass eine Erhöhung des Druckes in einem ringförmigen Zwischenraum zwischen Innenschiauch und Aussenschlauch nahezu ausschliesslich zu einer Dehnung des Aussenschlauches, nicht jedoch beispielsweise zu einer Schrumpfung des Innenschlauches führt, so dass letzterer seine Innenabmessung nahezu unverändert beibehalten kann.

Durch die Verringerung der Dicke des Materials des Aussenschlauches im Vergleich zu derjenigen des Innenschlauches ist es auf besonders einfache und wirtschaftliche Art möglich, den Aussenschlauch dehnfähiger und damit elastischer als den Innenschlauch auszugestalten.

Gemäss einer anderen Weiterbildung der Erfindung sind in einem ringförmigen Zwischenraum zwischen Innenkörper und Hüllenkörper mehrere vorzugsweise gleichmässig voneinander beabstandete, in Längsrichtung der Vorrichtung sich erstreckende Bänder vorzugsweise aus Metall angeordnet, wobei die Bänder vorzugsweise durch eine radiale Dehnung des Aussenschlauches gegen den Hüllenkörper pressbar sind. Die länglichen Bänder ermöglichen einerseits eine grosse Flexibilität, d.h. Biegbarkeit der erfindungsgemässen Vorrichtung, auf der andern Seite können sie auch dazu beitragen, dass die gesamte Vorrichtung weitgehend biegungsfest ausgebildet ist. Im Übrigen können die Bänder mit zu einer vorteilhaften, primären Steifigkeit der Vorrichtung beitragen, so dass diese leichter in einen Körpergang oder auch in eine Abzweigung eines Körpergangs einschiebbar ist. Vollkommen flexible Vorrichtungen sind nämlich weniger als solche mit einer gewissen Anfangssteifigkeit zum Einschieben in einen Körpergang geeignet. Die Anfangssteifigkeit könnte auch Initialsteifigkeit genannt werden. Dadurch ist die Einsetzbarkeit und die Handhabbarkeit der erfindungsgemässen Vorrichtung wirksam verbessert.

Gemäss einer vorteilhaften Weiterbildung der Erfindung ist der Hüllenkörper flexibel biegbar, jedoch torsionsfest ausgebildet und weist vorzugsweise ein Netz und/oder ein Gewebe und/oder wenigstens zwei gegenläufige Spiralen auf. Dadurch wird die flexible Handhabbarkeit der erfindungsgemässen Vorrichtung weiter verbessert. Diese Vorrichtung ist damit besonders verwindungssteif ausgebildet. Dies gilt umso mehr, als nicht nur, wie zuvor erwähnt, die Bänder sondern eben zusätzlich auch der Hüllenkörper die Verwindungssteifigkeit der Vorrichtung verbessern.

Vorteilhafterweise weist der Innenkörper zumindest auf der Innenseite seines Innenschlauches und/oder der HÜllenkörper zumindest auf seiner Aussenseite eine hydrophile Beschichtung auf. Somit bleibt auf der Innenseite und/oder Aussenseite der Vorrichtung ein vorteilhafterweise dünner Wasserfilm, wodurch die Gleitfähigkeit beispielsweise in einem Katheter oder in einem Gefäss, wie einem Körpergang, optimiert werden kann. Auch dadurch lässt sich die flexible Einsetzbarkeit und die Handhabbarkeit der erfindungsgemässen Vorrichtung weiter verbessern.

Gemäss einer anderen Weiterbildung der Erfindung ist der Innen körper durch einen Draht und/oder ein umfangsseitiges Geflecht versteift, wobei vorzugsweise der Draht und/oder das Geflecht aus Stahl oder einer Titan-Nickel-Legierung, vorzugsweise Nitinol®, gefertigt ist. Dadurch lässt sich beispielsweise der Innenkörper weiter stabilisieren, wobei der Draht beispielsweise wie eine Seele durch den Innenkörper verlaufen und vorteilhaft mit zu einer gewissen primären Steifigkeit des Innenkörpers und damit der gesamten Vorrichtung beitragen kann. Nitinol® ist ein Werkstoff mit einer gewissen "Gedächtnisfähigkeit", welche temperaturabhängig ist. Dieser Werkstoff wird beispielsweise für Stents gebraucht. Bei normaler Umgebungstemperatur ist dieser Stoff üblicherweise weich. Bei erhöhter Temperatur kann er hart werden.

Gemäss einer anderen Weiterbildung der Erfindung verjüngt sich der Aussendurchmesser der Vorrichtung zu deren vorderem Ende hin vorzugsweise stufenlos. Dadurch kann die Einsetzbarkeit bzw. Handhabbarkeit der erfindungsgemässen Vorrichtung weiter verbessert sein, da es möglich ist, dass dadurch auch enge oder schwer zugängliche Gefässe oder Körpergänge oder Abzweigungen derartiger Gefässe und Körpergänge mittels der Vorrichtung erreicht werden können.

Ausführungsbeispiele des Erfindungsgegenstandes werden nachfolgend anhang der Zeichnung näher erläutert, wobei alle beschriebenen und/oder bildlich dargestellten Merkmale für sich oder in beliebiger Kombination den Gegenstand der vorliegenden Erfindung unabhängig von ihrer Zusammenfassung in den Ansprüchen oder deren Rückbeziehung bilden, deren einzige Figur zeigt:
einen schematischen Querschnitt durch eine medizinische Vorrichtung zum zumindest teilweisen Einführen in einen Körpergang.

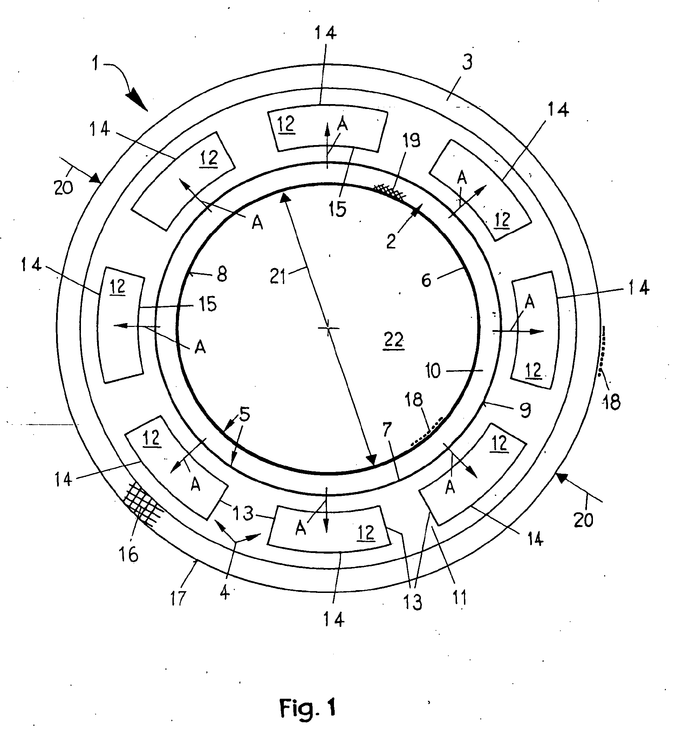

In Fig.1 ist schematisch ein Querschnitt durch eine medizintechnische Vorrichtung 1 zum zumindest teilweisen Einführen in einen nicht näher gezeigten Körpergang dargestellt.

Die medizintechnische Vorrichtung 1, nachfolgend kurz Vorrichtung genannt, hat einen länglichen, schlauchförmigen, flexiblen Innenkörper 2 sowie einen diesen zumindest abschnittsweise umfangsseitig umgebenden, länglichen, äusseren Hüllenkörper 3. Der besseren Übersicht halber sind in der einzigen Figur die einen Schnitt symbolisierenden Schraffuren weggelassen.

Ferner hat die Vorrichtung 1 eine Einrichtung 4 zum Überführen der Vorrichtung von einem flexiblen in einen versteiften und bedarfsentsprechend gebogenen Zustand und umgekehrt.

Erfindungsgemäss ist der Innenkörper 2 in Form eines Doppelschlauches 5 mit einem Innenschlauch 6 und einem Aussenschlauch 7 ausgebildet. Der Innenschlauch 6 bildet eine Innenwand 8 des Innenkörpers 2; der Aussenschlauch 7 bildet eine Aussenwand 9 des Innenkörpers 2, wobei der Aussenschlauch 7 den Innenschlauch 6 umfangsseitig etwa konzentrisch umgibt.

Die Einrichtung 4 zum Überführen der Vorrichtung 1 von einem flexiblen in einen versteiften Zustand und umgekehrt ist bei dem in der Figur gezeigten Ausführungsbeispiel dadurch gebildet, dass sich der Aussenschlauch 7 durch Erhöhung des Druckes in einem ringförmigen Zwischenraum 10 radial in Richtung der Pfeile A dehnt und dadurch einen Druck den Hüllenkörper 3 ausübt.

Gemäss einer bevorzugten Ausführungsform der Erfindung ist der Aussenschlauch 7 aus einem Material gefertigt, das dehnfähiger als dasjenige des Innenschlauches 6 ist. Diese erhöhte Dehnfähigkeit des Materials des Aussenschlauches 7 kann beispielsweise dadurch erreicht werden, dass die Dicke des Aussenschlauches 7 geringer als diejenige des Innenschlauches 6 ist.

Wie in der Figur dargestellt, sind in einem ringförmigen Zwischenraum 11 zwischen die Längsbänder und dann auf den Innenkörper 2 und Hüllenkörper 3 in Umfangsrichtung mehrere vorzugsweise gleichmässig voneinander beabstandete Bänder 12 angeordnet. Die Bänder 12 erstrecken sich vornehmlich in Längsrichtung der Vorrichtung 1 und sind vorzugsweise aus Metall gefertigt. Durch die radiale Dehnung des Aussenschlauches 7 in Richtung der Pfeile A sind die Bänder 12 gegen den Hüllenkörper 3 pressbar, wodurch die Steifigkeit auch in gebogenem Zustand der gesamten Vorrichtung 1 erhöht wird. Die radiale Dehnung des Aussenschlauches 7 kann durch Einbringen eines unter Druck stehenden Fluides, wie zum Beispiel einer Flüssigkeit oder eines Gases, in den ringförmigen Zwischenraum 10 des Innenkörpers 2 erfolgen.

Die Bänder 12 können einen geringen gegenseitigen Abstand voneinander aufweisen. Einander gegenüber liegende Kanten 13 der Bänder können parallel zueinander oder vorzugsweise nach innen hin konisch spitz zulaufen. Ebenso können die Aussenkanten 14 der Bänder 12 gekrümmt und in ihrem Radius an denjenigen des Hüllenkörpers 3 angepasst sein oder geradlinig verlaufen. Analoge Ausführungen gelten auch für die Innenkanten 15 der Bänder 12.

Gemäss einer besonders bevorzugten Ausführungsform der Erfindung ist der Hüllenkörper 3 flexibel biegbar, jedoch torsionsfest ausgebildet. Dazu weist der Hüllenkörper 3 ein Netz 16 und/oder ein Gewebe und/oder wenigstens zwei nicht näher gezeigte gegenläufige Spiralen auf. Der Hüllenkörper 3 kann selber netzartig ausgebildet sein oder in seinem Inneren ein solches Netz oder ein Gewebe aufweisen. In der Figur ist die Struktur des Netzes 16 lediglich an einer Stelle schematisch angedeutet.

Gemäss einer anderen Ausführungsform der Erfindung weist der Innenkörper 2 zumindest auf der Innenwand 8 seines Innenschlauches 6 und/oder der Hüllenkörper 3 zumindest auf seiner Aussenseite 17 eine hydrophile Beschichtung 18 auf, welche in der Figur ebenfalls lediglich jeweils an einer Stelle schematisch angedeutet ist. Ebenso ist die hydrophile Beschichtung 18 an der Innenwand 8 des Innenschlauches 6 lediglich schematisch gezeigt.

Gemäss einer weiteren bevorzugten Ausführungsform der Erfindung ist der Innenkörper 2 durch einen Draht und/oder ein umfangsseitiges Geflecht 19 versteift. Diese Versteifung kann, wie in der Figur schematisch angedeutet, im ringförmigen Zwischenraum 10 des Innenkörpers 2 angeordnet sein. Es ist aber auch möglich, das Geflecht an der Innenwand 6 und/oder an der Aussenwand 9 des Innenkörpers 2 vorzusehen.

Der nicht näher gezeigte Draht und/oder das Geflecht 19 sind vorzugsweise aus Stahl oder einer Titan-Nickel-Legierung, vorzugsweise aus Nitinol®, gefertigt.
Gemäss einer anderen, nicht näher gezeigten Ausführungsform der Erfindung verjüngt sich der Aussendurchmesser 20 der Vorrichtung 1 zu deren vorderem Ende hin vorzugsweise stufenlos.

Die Vorrichtung 1 ist damit sowohl als Schleuse oder Katheter aber auch als so genannte Führungseinrichtung für einen Katheter einsetzbar, wobei der Innendurchmesser 21 des Innenkörpers 2 in dem letztgenannten Fall mindestens 1 bis 5 und mehr mm beträgt. Bei der letztgenannten Ausführungsform könnte also ein Katheter durch den Innenraum 22 des Innenkörpers 2 hindurch geschoben werden. Andererseits könnte die erfindungsgemässe Vorrichtung auch als so genannte Führungseinrichtung eingesetzt werden, indem ein Katheter beispielsweise über die Vorrichtung vorgeschoben wird. Es ist klar, dass in beiden Fällen die Dimensionen in der Figur sehr stark vergrössert dargestellt sind.

Nachfolgend wird der Einsatz der erfindungsgemässen medizintechnischen Vorrichtung beispielhaft erläutert.

Üblicherweise wird die Vorrichtung 1 in einen Körpergang, wie z.B. eine Arterie oder eine Vene, im nicht versteiften Zustand eingeschoben und in dem Körpergang bis an die gewünschte, zu untersuchende Stelle vorgeschoben. Dabei hat die Vorrichtung eine gewisse Primär- oder Grundsteifigkeit, welche beispielsweise durch die Bänder 12, das Netz 16 oder das Geflecht 19 ermöglicht wird.

Sofern die Vorrichtung mit Hilfe der Einrichtung zum Überführen der Vorrichtung von einem flexiblen in einen versteiften Zustand überführt werden soll, wird ein Druck an den Doppelschlauch 5 beispielsweise mittels eines Druckmediums, wie z.B. eine Flüssigkeit oder ein Gas, angelegt. Dieser Druck bewirkt eine Erhöhung des Aussendurchmessers des Doppelschlauchs 5, indem der Aussenschlauch 7 sich entlang der radial gerichteten Pfeile A nach aussen verschiebt. Dabei bleibt der Innendurchmesser 22 des Innenkörpers 2 weitgehend oder vollständig erhalten. Sofern vorhanden, können die vorgenannten Bänder 12 schliesslich mit Hilfe des sich radial nach aussen bewegenden Aussenschlauches 7 gegen den Hüllenkörper 3 gedrückt werden. Somit trägt die radiale Dehnung des Aussenschlauches 7 des Innenkörpers 2 zu einer Versteifung der gesamten medizintechnischen Vorrichtung 1 bei.

Die erfindungsgemässe Vorrichtung ist, wie zuvor erwähnt, sowohl als Schleuse oder Katheter als auch als Führungseinrichtung einsetzbar.

Die Krümmung bzw. der Radius der Aussenkante 17 jedes Bandes 12 kann an den Radius bzw. die Krümmung des Hüllenkörpers 3 angepasst sein. Ferner kann die Innenkante 15 dieser Bänder an den Aussendurchmesser des Innenkörpers 2 angepasst sein und letzterem entsprechen. Dies gilt sowohl für den flexiblen als auch für den versteiften Zustand der beschriebenen Vorrichtung.

Damit ist eine medizintechnische Vorrichtung geschaffen, welche vielseitiger einsetzbar und vorteilhafter handhabbar ist.

## Patentansprüche

1. Medizintechnische Vorrichtung zum zumindest teilweisen Einführen in einen Körpergang,
mit einem länglichen, schlauchförmigen, flexiblen Innenkörper (2),
einem diesen zumindest abschnittsweise umfangsseitig umgebenden, länglichen, äusseren Hüllenkörper (3) und
mit einer Einrichtung (4) zum Überführen der Vorrichtung (1) von einem flexiblen in einen versteiften Zustand und umgekehrt,
wobei der Innenkörper (2) in Form eines Doppelschlauches (5) mit einem eine Innenwand (8) des Innenkörpers (2) bildenden Innenschlauch (6) und einem eine Aussenwand (9) des Innenkörpers (2) bildenden, den Innenschlauch (6) umfangsseitig konzentrisch umgebenden Aussenschlauch (7) ausgebildet ist, und
die Einrichtung (4) zum Überführen der Vorrichtung (1) von einem flexiblen in einen versteiften Zustand und umgekehrt **dadurch** gebildet ist, dass sich der Aussenschlauch (7) durch Erhöhung des Druckes in einem ringförmigen Zwischenraum (10) zwischen Innenschlauch (6) und Aussenschlauch (7) radial dehnt und **dadurch** einen Druck auf den Hüllenkörper (3) ausübt,
**dadurch gekennzeichnet, dass**
in einem ringförmigen Zwischenraum (11) zwischen Innenkörper (2) und Hüllenkörper (3) mehrere in Längsrichtung der Vorrichtung (1) sich erstreckende Bänder (12) vorzugsweise aus Metall angeordnet sind und die Bänder (12) einander gegenüber liegende Kanten (13) aufweisen, die parallel zueinander oder nach innen hin konisch spitz zulaufen.

2. Medizintechnische Vorrichtung nach Anspruch 1, **dadurch gekennzeichnet, dass** die Aussenkanten (14) der Bänder gekrümmt und in ihrem Radius an denjenigen des Hüllenkörpers (3) angepasst sind und vorzugsweise auch die Innenkanten (15) der Bänder (12) gekrümmt und in ihrem Radius an denjenigen des Innenkörpers (2) angepasst sind.

3. Vorrichtung nach Anspruch 1 oder 2, **dadurch gekennzeichnet, dass** der Aussenschlauch (7) aus einem Material gefertigt ist, das dehnfähiger als dasjenige des Innenschlauches (6) vorzugsweise **dadurch** ist, dass die Dicke des Aussenschlauches (7) geringer als diejenige des Innenschlauches (6) ist.

4. Vorrichtung nach einem der Ansprüche 1 bis 3, **dadurch gekennzeichnet, dass** die Bänder (12) durch eine radiale Dehnung des Aussenschlauches (7) gegen den Hüllenkörper (3) pressbar sind.

5. Vorrichtung nach einem der vorhergehenden Ansprüche, **dadurch gekennzeichnet, dass** der Hüllenkörper (3) flexibel dehnbar, jedoch torsionsfest ausgebildet ist.

6. Vorrichtung nach Anspruch 5, **dadurch gekennzeichnet, dass** der Hüllenkörper (3) ein Netz (16) und/oder ein Gewebe und/oder wenigstens zwei gegenläufige Spiralen aufweist.

7. Vorrichtung nach einem der vorhergehenden Ansprüche, **dadurch gekennzeichnet, dass** der Innenkörper (2) zumindest auf der Innenwand (8) seines Innenschlauches (6) und/oder der Hüllenkörper (3) zumindest auf einer Aussenseite (17) eine hydrophile Beschichtung (18) aufweist.

8. Vorrichtung nach einem der vorhergehenden Ansprüche, **dadurch gekennzeichnet, dass** der Innenkörper (2) durch einen Draht und/oder ein umfangsseitiges Geflecht (19) versteift ist.

9. Vorrichtung nach Anspruch 8, **dadurch gekennzeichnet, dass** der Draht und/oder das Geflecht (19) aus Stahl oder einer Titan-Nickel-Legierung, vorzugsweise Nitinol®, gefertigt ist.

10. Vorrichtung nach einem der vorhergehenden Ansprüche, **dadurch gekennzeichnet, dass** sich der Aussendurchmesser (20) der Vorrichtung (1) zu deren vorderem Ende hin vorzugsweise stufenlos verjüngt.

## Claims

1. Medico-technical device for introducing at least partially into a body passage,
with an elongate, tubular, flexible internal body (2),
an elongate exterior enveloping body (3) which surrounds, at least partially, said internal body (2) on the periphery thereof, and
with a mechanism (4) for transferring the device (1) from a flexible state to a rigid state and vice-versa,
where the internal body (2) which is in the form of a double tube (5), comprises an inner tube (6) which forms an inner wall (8) of the internal body (2) and an external tube (7) which forms an external wall (9) of the internal body (2), said external tube (7) surrounding the inner tube (6) in a concentric manner on the periphery thereof, and
the mechanism (4) for transferring the device (1) from a flexible state into a rigid state and vice-versa is embodied in such a manner that the external tube (7) extends radially by increasing the pressure in an annular-shaped intermediate chamber (10) between inner tube (6) and outer tube (7) and thus exerts pressure on the enveloping body (3);
**characterised in that**
in an annular intermediate chamber (11) between the internal body (2) and the enveloping body (3) several bands (12), preferably of metal, are arranged extending longitudinally along the device (1), and the bands (12) have facing edges (13) running parallel to each other or converging at a conical angle towards the inside.

2. Medico-technical device according to Claim 1, **characterised in that** the outer edges (14) of the bands are curved and their radius chosen to match that of the external body (3), and preferably the inner edges (15) of the bands (12) are also curved and their radius chosen to match that of the internal body (2).

3. Device according to Claim 1 or 2, **characterised in that** the outer tube (7) is made of a material which is more extensible than that of the inner tube (6), preferably by the thickness of the outer tube (7) being less than that of the inner tube (6).

4. Device according to one of the Claims 1 to 3, **characterised in that** the bands (12) can be pressed against the enveloping body (3) by a radial extension of the outer tube (7).

5. Device according to one of the aforegoing Claims, **characterised in that** the enveloping body (3) is made so as to be flexibly extensible, yet firm against torsion.

6. Device according to Claim 5, **characterised in that** the enveloping body (3) has a net (16) and/or a fabric and/or at least two spiral coils running in opposite directions.

7. Device according to one of the aforegoing Claims, **characterised in that** the internal body (2) has a hydrophilic coating (18) at least on the inner wall (8) of its inner tube (6), and/or the enveloping body (3) has a hydrophilic coating (18) at least on an outside surface (17).

8. Device according to one of the aforegoing Claims, **characterised in that** the internal body (2) is stiffened by a wire and/or a mesh (19) around its periphery.

9. Device according to Claim 8, **characterised in that** the wire and/or the mesh (19) is made of steel or a titanium-nickel alloy, preferably Nitinol®.

10. Device according to one of the aforegoing Claims, **characterised in that** the outside diameter (20) of the device (1) tapers down towards its front end preferably in a smooth gradation.

## Revendications

1. Instrument de technique médicale pour introduction au moins partielle dans un canal corporel,
comportant un corps intérieur souple (2), oblong, en forme de tuyau flexible,
un corps extérieur (3) formant enveloppe, oblong, qui entoure ce dernier sur sa circonférence, au moins par sections, et
un dispositif (4), pour faire passer l'instrument (1) d'un état souple à un état rigidifié et inversement,
dans lequel le corps intérieur (2) est configuré sous forme d'un tuyau flexible double (5), avec un tuyau flexible intérieur (6) formant une paroi intérieure (8) du corps intérieur (2) et un tuyau flexible extérieur (7), formant une paroi extérieure (9) du corps intérieur (2), entourant d'une manière concentrique côté circonférence le tuyau flexible intérieur (6), et
le dispositif (4) pour faire passer l'instrument (1) d'un état souple à un état rigidifié et inversement étant formé par le fait que le tuyau flexible extérieur (7) subit une dilatation radiale sous l'effet d'une augmentation de la pression dans un espace intérieur annulaire (10) entre le tuyau flexible intérieur (6) et le tuyau flexible extérieur (7), et de ce fait exerce une pression sur le corps (3) formant enveloppe,
**caractérisé en ce que**
plusieurs bandes (12), de préférence en métal, s'étendant dans la direction longitudinale de l'instrument (1), sont disposées dans un espace intermédiaire annulaire (11) entre le corps intérieur (2) et le corps (3) formant enveloppe, et les bandes (12) présentent des arêtes (13), opposées l'une à l'autre, qui courent parallèlement l'une à l'autre, en présentant une conicité vers l'intérieur.

2. Instrument de technique médicale selon la revendication 1, **caractérisé en ce que** les arêtes extérieures (14) des bandes sont incurvées, et leur rayon est adapté à celui du corps (3) formant enveloppe, et de préférence aussi les arêtes intérieures (15) des bandes 12 sont incurvées, et leur rayon est adapté à celui du corps intérieur (2).

3. Instrument selon la revendication 1 ou 2, **caractérisé en ce que** le tuyau flexible extérieur (7) est fabriqué en un matériau qui de préférence a un pouvoir de dilatation plus grand que celui du tuyau flexible intérieur (6), du fait que l'épaisseur du tuyau flexible extérieur (7) est inférieure à celle du tuyau flexible intérieur (6).

4. Instrument selon l'une des revendications 1 à 3, **caractérisé en ce que** les bandes (12) peuvent être pressées contre le corps (3) formant enveloppe, sous l'effet d'une dilatation radiale du tuyau flexible extérieur (7).

5. Instrument selon l'une des revendications précédentes, **caractérisé en ce que** le corps (3) formant enveloppe est configuré souple et dilatable, mais résistant à la torsion.

6. Instrument selon la revendication 5, **caractérisé en ce que** le corps (3) formant enveloppe comporte un filet (16) et/ou un tissu et/ou au moins deux spirales tournant en sens inverse.

7. Instrument selon l'une des revendications précédentes, **caractérisé en ce que** le corps intérieur (2) comprend un revêtement hydrophile (18) au moins sur la paroi intérieure (8) de son tuyau flexible intérieur (6), et/ou le corps (3) formant enveloppe comprend un revêtement hydrophile (18) au moins sur une face extérieure (17).

8. Instrument selon l'une des revendications précédentes, **caractérisé en ce que** le corps intérieur (2) est rigidifié par un fil métallique et/ou un treillis (19) côté circonférence.

9. Instrument selon la revendication 8, **caractérisé en ce que** le fil métallique et/ou le treillis (19) sont fabriqués en acier ou en un alliage de titane et de nickel, de préférence en Nitinol^{®}.

10. Instrument selon l'une des revendications précédentes, **caractérisé en ce que** le diamètre extérieur (20) de l'instrument (1) se rétrécit, de préférence d'une manière continue, vers son extrémité avant.
